# EUROPEAN PATENT SPECIFICATION

(11) **EP 1 500 376 B1**
(45) Date of publication and mention of the grant of the patent: **14.03.2007**
(21) Application number: 04016796.7
(22) Date of filing: 16.07.2004
(51) Int. Cl.: A61B 17/70, A61B 17/80

(54) **Cervical spine fixator and screwdriver used therefor**
Halswirbelstabilisierungsvorrichtung und Schraubenzieher dafür
Dispositif de stabilisation des vertèbres cervicales et tournevis pour cela

(30) Priority: 22.07.2003 KR 2003050152
(43) Date of publication of application: 26.01.2005
(73) Proprietor: U & I Corporation, Kyunggi-do 480-080 (KR)
(72) Inventor: Koo, Ja-Kyo, Seoul 139-220 (KR)
(74) Representative: Melzer, Wolfgang

(56) References cited:
- EP-A- 1 169 971
- DE-A- 4 409 833
- US-A1- 2001 041 894
- US-A1- 2002 173 790
- US-B1- 6 361 537
- US-B1- 6 402 755

## Description

### FIELD OF THE IN VENTION

The present invention relates generally to a cervical spine fixator as an anterior cervical implant used for treating and correcting cervical disc diseases, cervical hernial disc diseases, cervical spondylosis myelopathy, cervical fractures, dislocations, tumors and cervical kyphotic deformity in orthopedics and neurosurgical fields.

### BACKGROUND OF THE INVENTION

In general, cervical spine disorders are treated by holding respective cervical vertebrae in desired spatial relationships and orientations relative to each other. One common device for spinal fixation includes a plate and a fixation device comprising a plurality of screws for being fixedly inserted to the plate.

The fixation device includes fixation screws for being driven or fastened into the upper and lower cervical vertebrae of the cervical spine, whereby respective vertebrae are fixed and treated by the locking force of the plates and the fixed screws.

A surgical operation by the fixation device is conducted as follows. A surgeon makes an incision into a patient's affected part of the cervical vertebrae. Next, while the plate of the fixation device is adjoined to the affected part, the screw is inserted into the front surface of the plate to allow the fixation device to be fixed to the cervical vertebrae positioned at the upper and lower ends of the affected part.

However, there is a drawback associated with this technique in that screws or other fasteners tend to gradually become loose after fixation. Receiving a slight shock or vibration due to walking, climbing stairs or engaging in a vigorous activity following treatment increases this tendency, jeopardizing the integrity of the fixation. Moreover, as the fasteners are loosened, the outward protrusion of the heads over other components of the fasteners can be a source of discomfort and potentially cause trauma to adjacent and surrounding soft tissue, resulting in a deformity of the affected part.

There have been attempts to solve the above drawback. For example, an auxiliary plate hitching the head of the screw is locked by a fastening member after the screw is fixed, or devices such as a cam locking system and the like for preventing the screws from backing out have been developed.

However, there is a drawback in the structure of fixing the auxiliary plate by the fastening member to thereby prevent the screws from backing out according to the prior art thus described in that the auxiliary plate can become loose due to the unscrewing of the fastening member. There is still another drawback in that it is inconvenient to conduct an operation due to the re-fixation of a separate fastening member after the fixation of the screws. There is still a further drawback in that the cam locking system is too complicated in structure thereof

Meanwhile, other attempts have been disclosed in WO 00/78238 A1 and EP 1 169 971 A2, where a member elastically deformed by the effect of pressure is used to fix a head of a screw. According to WO 00/78238 A1, at least one retaining means is provided which can be elastically deformed under the effect of pressure enabling a screw to pass into and be installed in a bore hole, whereby the retaining means returns to its position of origin and is not deformed when the effect of the pressure is withdrawn, being positioned above a head of the screw. The retaining means is received into a separate bore adjacent to the bore hole accommodating the screw.

EP 1 169 971 A2 discloses an implant according to the preamble of claim 1, particularly for the spinal column, comprising a joining member such as a plate exhibiting openings or orifices, bone-anchoring members such as bone screws capable of being accommodated in the orifices and at least one split ring capable of holding the members in the orifices. The split ring can come into direct contact with the anchoring member or members to hold the member or members in the orifices.

However, there are drawbacks in WO 00/78238 A1 and EP 1 169 971 A2 which represent the closest prior art in that the retaining means or split ring is accommodated in the orifices of the plate by a member elastically deformed by the effect of pressure for fixing of a screw head, making the fixation process more difficult, creating more difficulty in manufacturing, and resulting in a thickened plate. When the plate is thickened, a patient may feel a foreign substance after a surgical Operation. Thus, it has been suggested by patients and surgeons that the plate be thinned to remove the discomfort and the cervical spine fixator be simple in operation to reduce the operating time. A surgical plate is known from D1. A surgical plate is fixed to a spine of a patient by use of several screws that are inserted into vertebrae. The screws are prevented from loosening by a pawl plate with torsion bars that are pushed aside by a toothed outer circumferential edge of the screw head. When such a tooth has passed the torsion bars return to their original position thereby preventing the screws from being rotated in the opposite direction. The pawl plate is located in a recess of the surgical plate.

### SUMMARY OF THE INVENTION

The present invention is disclosed to meet the above-mentioned requirements and it is an object of the present invention to provide a cervical spine fixator that is easier to manufacture and a plate that is reduced in thickness, thus decreasing the feeling of a foreign substance after a surgical operation with the screw head fixture member secured in a recess of the plate.

It is another object of the present invention to provide a screwdriver for a cervical spine fixator adapted to be convenient for surgery and to shorten the operating time.

The cervical spine fixator according to one embodiment of the present invention comprises: a plate for being adjoined to a cervical spine fixation region; and a plurality of screws for insertion and fixation in the cervical spine and simultaneously coupled to the plate. An adjacent region coupled with the screws in the plate is fixed with a screw head fixture member having a protruder, which is deformed under the effect origin pressure by a screw head when the screw is inserted and returns to its position of origin when the screw head is completely accommodated into the plate. Said plate is formed thereon with a receiving groove for accommodating the screw head and the screw head fixture member. The receiving groove is formed with openings into which the screws are inserted and a recess into which the screw head is received. The distal tip ends of said protruder are formed with a hitching protruder. The recess of said plate is formed with a hitching hole for passing through the thickness of said plate for accommodating said hitching protruder. The opposite end of the screw head fixture member is fixed to said plate. The screw head fixture member is an elastic plate protrusively formed with the protruder.

Preferably, the screw head fixture member is a U-shaped elastic ring functioning as the protruder. Preferably, the distal tip ends of the protruder or the elastic ring bend towards mutually opposite directions. Preferably, the protruder or the U-shaped elastic ring is tapered-off in the cross-section thereof towards the opposite side of the distal tip end so as to be easily deformed.

Preferaby, the distal tip ends of the protruder or the U-shaped elastic ring is formed with a hitching protruder, and the recess of the plate is formed with a hitching hole through which the plate passes.

Furthermore, the mid-section of the plate is formed with a window-shaped hole to observe a cage between the vertebrae during surgery, and the window-shaped hole is formed with threads into which a screw is threaded for fixing the cage.

The screw head has a cornical-shaped incline plane configuration so that the protruder or the U-shaped elastic ring can smoothly slide to be elastically deformed under the effect of pressure when the screws are inserted into the plate.

The protruder of the screw head fixture member or an opposite end of the distal end at the U-shaped elastic ring is fixed to the plate by a rivet.

The upper surface of the screw head is grooved in the shape of a straight line or a cross, and the grooved surface is deepened towards the center thereof to form a concaved curvature so that the protruder or the U-shaped elastic ring can be deformed under the effect of pressure by the tip end of a screwdriver when the screws are installed or removed.

The distal tip end of the screwdriver for rotating the screw is protrusively formed with a protruder in the shape of a straight line or a cross so as to be inserted into the groove, whereas the protruder is formed in a convex shape for being adjoined onto the concave curvature of the screw head.

### BRIEF DESCRIPTION OF THE DRAWINGS

For a better understanding of the nature and objects of the present invention, reference should be made to the following detailed description with the accompanying drawings, in which:
FIG. 1 is a perspective view for illustrating a cervical spine fixator according to state of the art;
FIG. 2 is an exploded perspective view of FIG. 1;
FIG. 3 is a cross-sectional view taken along line A-A of FIG 1;
FIG. 4 is a plan for illustrating a deformed state of a screw head fixture member when a screw is inserted;
FIG. 5 is a cross-sectional view for illustrating a screw head fixture member hitching a screw head when a screw is completely inserted;
FIG. 6 is a perspective view for illustrating another cervical spine fixator according to state of the art;
FIG. 7 is a cross-sectional view taken along the arrow B-B of FIG. 6;
FIG. 8 is a perspective view of a cervical spine fixator according to the present invention;
FIG. 9 is an exploded perspective view of FIG. 8;
FIG 10 is a bottom view taken from a lower side of the cervical spine fixator of FIG. 8; and
FIG. 11 is a perspective view for illustrating a screwdriver used for a cervical spine fixator during surgery according to the present invention.

### DETAILED DESCRIPTION OF THE PREFERRED EMBODIMENT

The preferred embodiments of the present invention will be described in detail with reference to the annexed drawings, where the present embodiment are not limiting the scope of the present invention but are given only as an illustrative purpose.

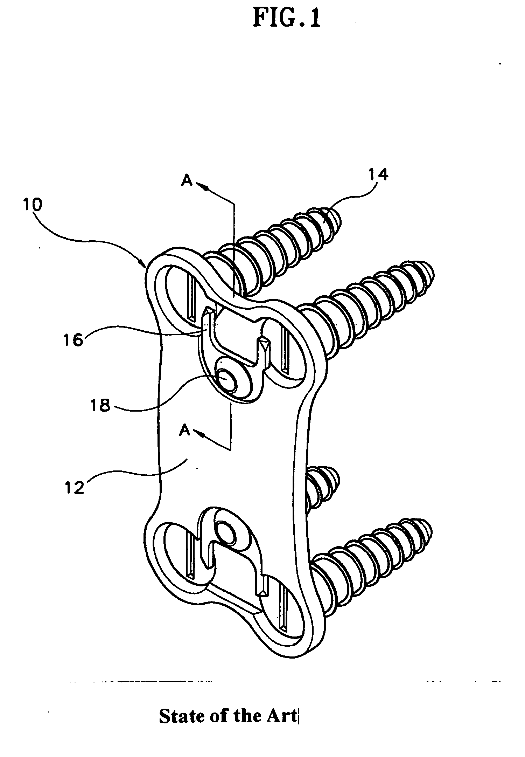

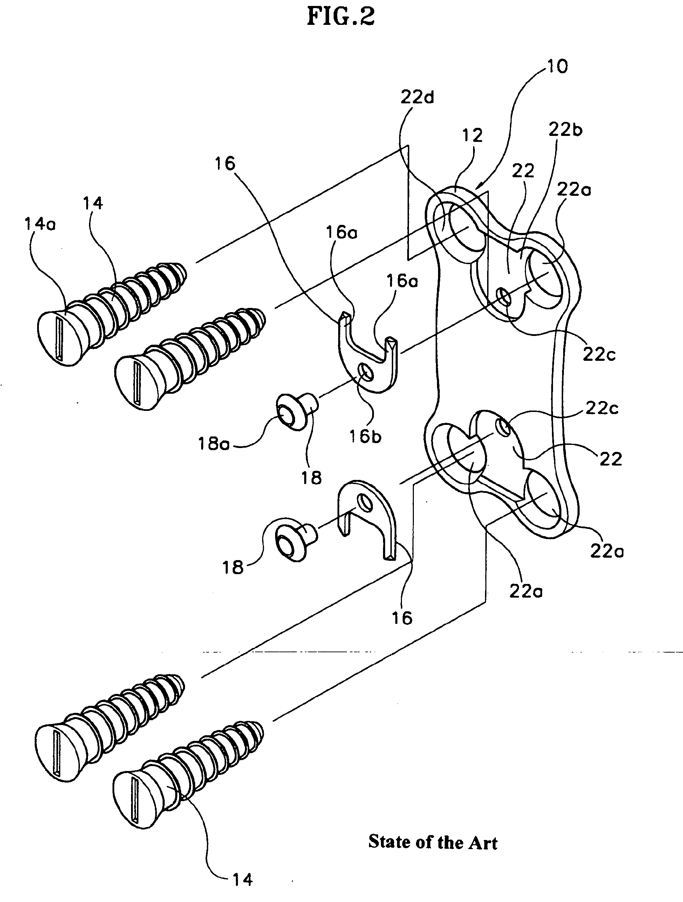

As shown in FIGS. 1 and 2, a cervical spine fixator (10) known from the state of the art includes a plate (12) for being adjoined to a cervical spine fixture region, a plurality of screws coupled to the plate (12) and simultaneously fastened to the cervical spine, and a screw head fixture member (16) fixed to the plate (12) for preventing the screws from backing out.

The screw head fixture member (16) is fixed to the plate (12) by way of a well-known attachment method such as riveting (18) or welding. Generally, the plate (12) has a rectangular shape and is bent to conform to the curvature of the cervical spine. Receiving grooves are formed on the surface of the plate (12) opposite to the surface that is affixed to the cervical spine, for accommodating the head of the screw (14) and the screw head fixture member (16),

The receiving grooves (22) are formed at both ends of the plate (12), and each receiving groove (12) has an approximate T-shape when viewed from the top of the plate (12). Both lateral ends of the groove (22) are formed with openings (22a, 22a) for insertion of the screws. The vertical portion of the T-shaped groove (22) is formed with a recess (22b) for accommodating the screw head fixture member (16), and the recess (22b) has a rivet hole (22c) at one end thereof for insertion of a rivet (18) for fixing the screw head fixture member (16).

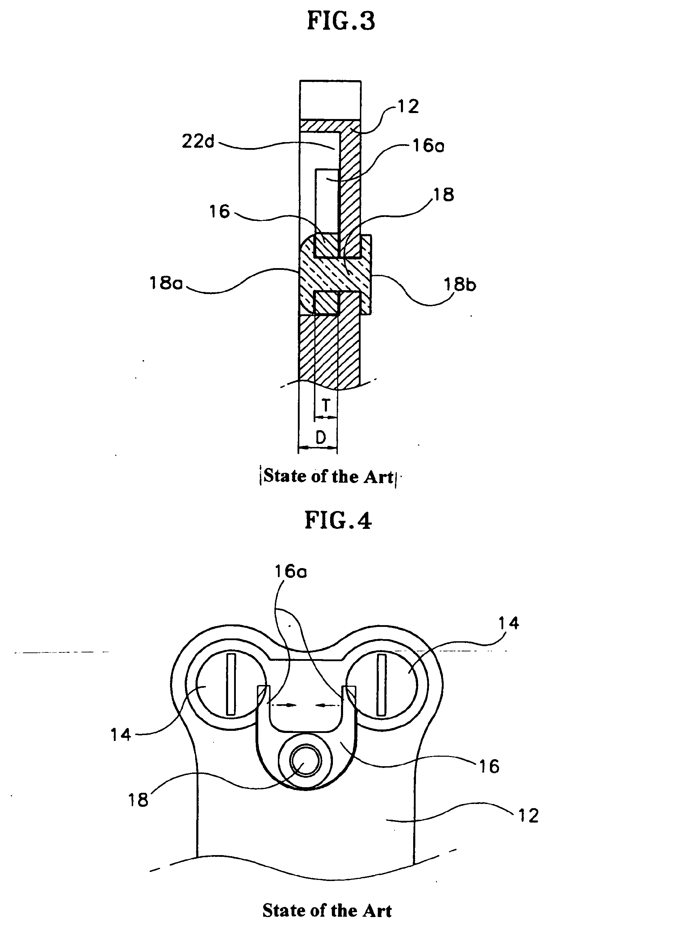

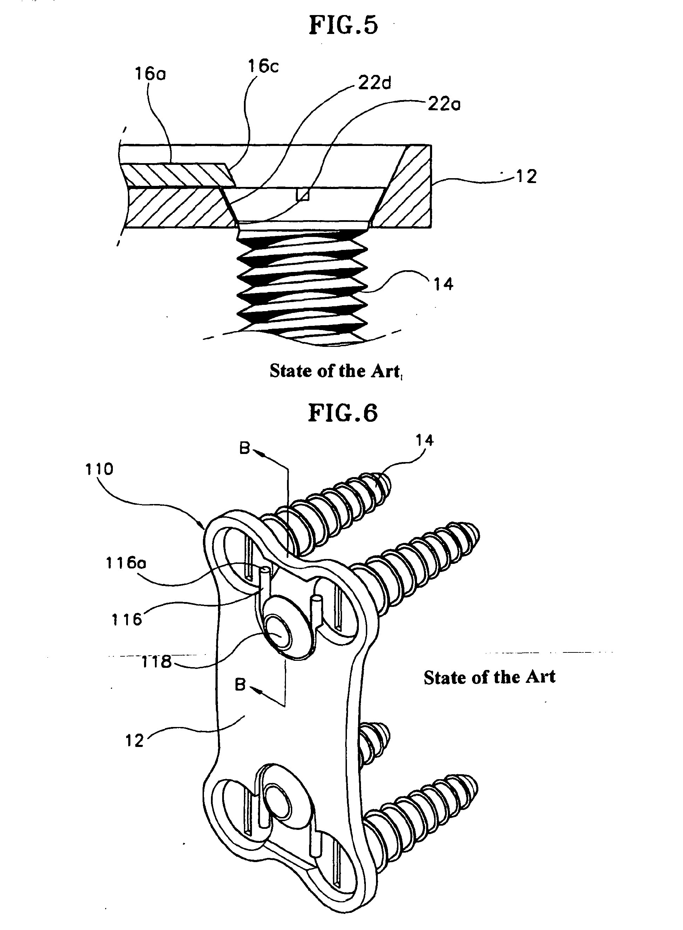

Furthermore, as illustrated in FIG. 3, the depth (D) of the recess (22b) is equal to or deeper than the thickness (T) of the screw head fixture member (16) such that a head (18a) of the rivet (18) does not protrude out of the plane surface of the plate (12). As shown in FIG. 5, the opening (22a) of the receiving groove (22) is formed with an incline (22d) to allow a head of the screw (14) to be hitched thereat.

The four screws (14) are members inserted into four holes formed at the plate (12), and the head of each screw (14) is formed with an incline (14a) for easily deforming protruders (described later) of the screw head fixture member (16) when the screw (14) is inserted.

The screw head fixture member (16) is an elastic horse shoe-shaped plate having protruders (16a) each hitching the heads of the screws (14). The screw head fixture member (16) is deformed under the effect of pressure by the incline (14a) of the heads of the screws when the screws are inserted and returns to its position of origin when the heads of the screws (14) are completely accommodated into the incline (22d) formed at the hole of the plate (12). The protruder (16a) of the screw head fixture member (16) is formed at the other side thereof with a rivet hole (16b) so that the rivet (18) is fixedly inserted into the plate (12). Each protruder (16a) is like a prong of a horse shoe magnet.

As seen in FIG. 5, the distal end of each protruder (16a) is formed with an incline (16c) for allowing the incline (14a) of the screw to smoothly slide when the head of the screw (14) is inserted and for prompting the protruder (16a) to be easily deformed. The protruder (16a) tapers off towards the opposite side of the distal end so as to be easily deformed during surgery.

The rivet (18) is inserted into the rivet hole (16b) of the screw head fixture member and the rivet hole (22c) of the plate, and as seen in FIG 3, the rivet (18) forms a heading (18b) at the surface of the plate (12) that is affixed to the cervical spine to thereby fix the screw head fixture member (16).

In the cervical spine fixator shown in figs. 1 to 5 the screw (14) is inserted into the opening (22a) of the plate (12), where the head of the screw (14) is accommodated into the incline (22d) of the hole (22a) to slide along the incline (16c) formed at the protruder (16a) of the screw head fixture member (16), and as shown in FIG. 4, the head of the screw (14) pushes the protruder (16a) in the arrow direction.

Furthermore, when the head of the screw (14) is completely fitted at the incline (22d), the protruder (16a) returns to its original position to be hitched by the upper surface of the head of the screw (14) as shown in FIG. 5, thus preventing the screw from being released or loosened.

FIG 6 is a perspective view for illustrating another cervical spine fixator known from the state of the art.

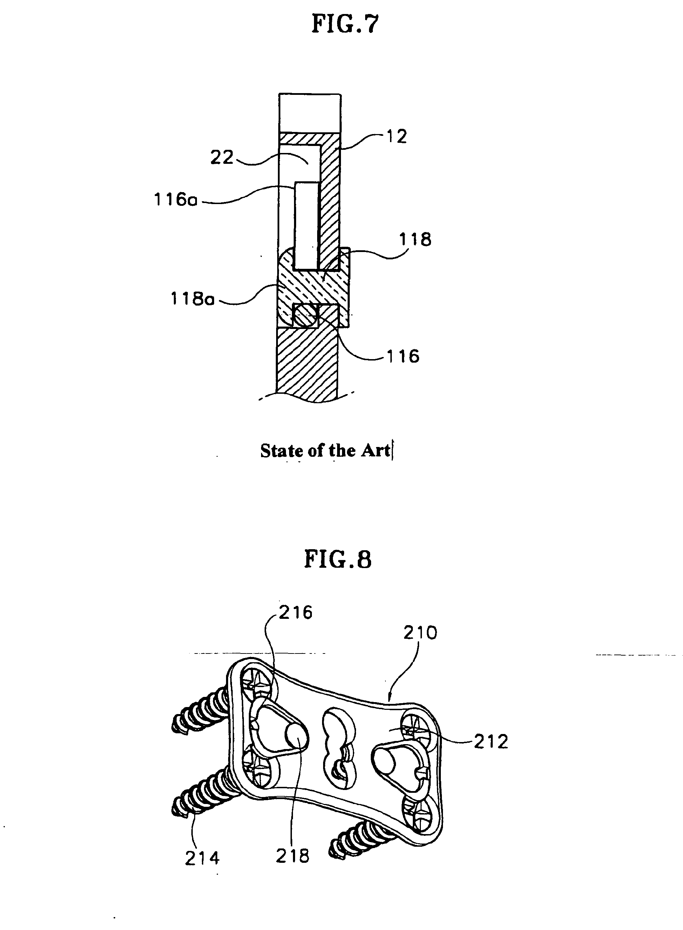

The cervical spine fixator (110) includes a screw head fixture member (116) of a U-shaped ring, and both ends (116a) of the U-shaped ring function as the protruders. The rivet (118) has a head large enough to press the curvature of the U-shaped ring. FIG. 7 is a cross-sectional view taken along an arrow of FIG.6. Other constructions of the second known fixator are the same as those of the first known fixator such that further explanation will not be necessary.

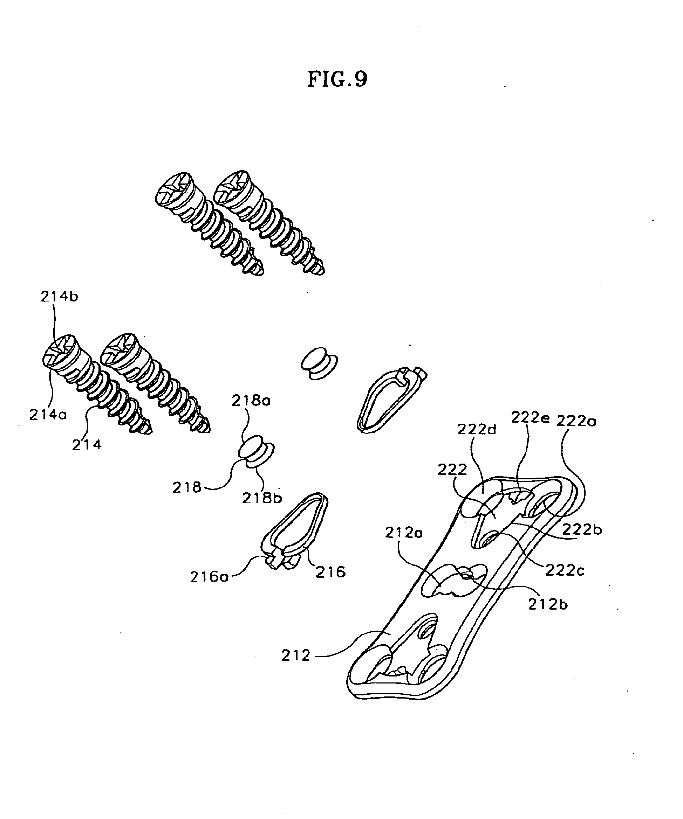

FIGS. 8 and 9 are respectively a perspective view and an exploded perspective view of a cervical spine fixator according to the present invention, wherein the cervical spine fixator (210) includes a plate (212) for being attached to a cervical spine fixture region, a plurality of screws (214) coupled to the plate (212) and simultaneously fixedly inserted into the cervical spine, and a screw head fixture member (216) fixed to the plate (212) for preventing the screws (214) from being loosened. The screw head fixture member (216) is fixed to the plate (212) by way of a well-known coupling method such as riveting (218), welding or the like.

The plate (212) has a near rectangular shape and is bent to conform to the curvature of the cervical spine. The surface of the plate (212) opposite to the surface that is affixed to the cervical spine is formed with receiving grooves (222) for accommodating the screw head fixture member (216) and heads of the screws (214).

The receiving grooves (222) are formed at both ends of the plate (212), and each receiving groove (222) has an approximate T-shape when viewed from the top of the plate (212). Both lateral ends of each T-shaped groove (222) are formed with openings (222a, 222a) for insertion of the screws. The vertical portion of each T-shaped groove (222) is formed with a recess (222b) for accommodating the screw head fixture member (216), and the recess (222b) is defined at one side thereof with a rivet hole (222c) for insertion of a rivet (218) for fixing the screw head fixture member (16). The structure of accommodating the screw head fixture member (216) into the recess (222b) can markedly reduce the thickness of the plate (212), actually up to a 1.6mm.

The depth (D) of the recess (222b) is equal to or deeper than the thickness (T) of the screw head fixture member (216) such that the head (218a) of the rivet (218) does not protrude out of the plane surface of the plate (212) as shown in FIG 3. Furthermore, the opening (222a) of the receiving groove (222) is formed with an incline (222d) to allow the head of each screw (214) to be hitched thereat as shown in **FIG. 5.**

A hitching hole is formed between the two openings (222a, 222a) in the recess (222b) where a hitching protruder of the screw head fixture member (216) can be hitched thereat. The hitching hole (222e) that passes through the thickness of the plate (212). The width of the recess (222b) becomes narrowed from the hitching hole (222e) towards the rivet hole (222c).

Furthermore, the mid-section of the plate (212) is formed with a window-shaped hole (212a) to observe a cage between the vertebrae during surgery, and the window-shaped hole (212a) is a long hole in the width direction of the plate. The mid-section of the window hole (212a) is wasp-waisted, where a screw thread (212b) is formed with threads into which a screw (not shown) is threaded for fixing the cage.

The screws (214) are members respectively driven into the cervical spine by being inserted into four holes each formed at the plate (212).

The screw head is laterally formed with a conical incline (214a) into which the screws (214) are inserted, and the distal end of the U-shaped elastic ring (described later) of the screw head fixture member (216) can be easily deformed.

The upper surface of the screw head is formed with a crossed groove (214b), and the crossed groove (214b) becomes deepened towards the center thereof to form a concaved curvature so that the distal end of the U-shaped elastic ring (described later) of the screw head fixture member (216) can be deformed under the effect of pressure by the tip end of a screwdriver when the screws (214) are installed or removed. The upper surface of the screw head may be formed with a straight groove, which becomes deepened towards the center thereof

The screw head fixture member (216) is a U-shaped elastic ring which functions as the protruders at both ends thereof, and a gap between both ends is narrowed towards the opposite side of the distal end to fit into the width of the recess (222b) of the receiving groove. The distal ends of the screw head fixture member (216) of the U-shaped elastic ring are bent towards each other, and each bent end is formed with a hitching protruder (216a), which hitchingly fits into the hitching hole (222e).

The screw head fixture member (216) is deformed under the effect of pressure by the incline (214a) of the screw head in relation to the insertion of the screw (214) and returns to its position of origin to get hitched at the head of the screw (214) when the head of the screw (214) is completely accommodated into the incline (222d) formed at the hole of the plate (212), where the thickness or cross-section becomes smaller from one distal end of the screw head fixture member (216) to the opposite end to expedite the deformation.

The rivet (218) is hitched at an inner central portion of the screw head fixture member (216) and is simultaneously inserted into the rivet hole (222c) of the plate, and the rivet (218) is formed with a heading (218b) at a surface of the plate (212) for being attached to the cervical spine to thereby fix the screw head fixture member (216).

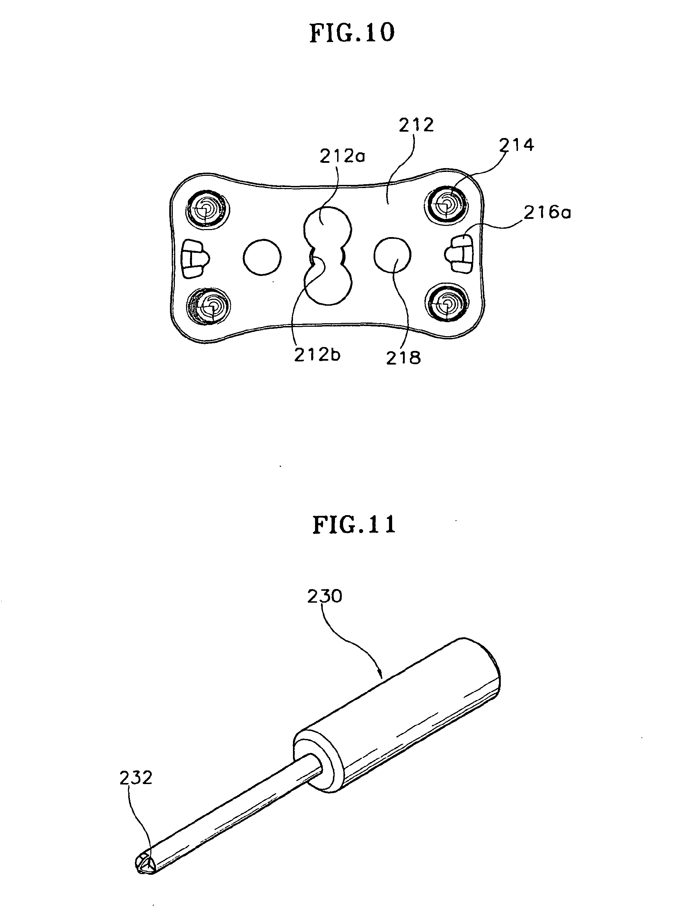

The screw head fixture member (216) is fixed by the rivet (218) after the hitching protruder (216a) is inserted into the hitching hole (222e) of the plate (212). The hitching protruder (216a) of the screw head fixture member (216) hitches and protrudes toward the surface adjoining the cervical spine at the plate (212) as shown in FIG. 10.

As a result, even if the screw becomes loose by the activity of a patient, the hitching protruder (216a) is hitched by the hitching hole (222e) of the plate (212) to prevent the screw head fixture member (216) from being protrusively deformed, thereby preventing irritation in the affected area.

In the cervical spine fixator thus described according to the present invention, the screw (214) is inserted into the opening (222a) of the plate (212) and thereafter inserted into the cervical spine. The head of the screw (214) is correspondingly fitted into the incline (222d) of the hole (222a) while pushing the distal end of the screw head fixture member inward in the process.

Furthermore, when the head of the screw (214) is fully accommodated into the incline (222d), the distal end of the screw head fixture member (216) returns to its position of origin to be hitched at the upper surface of the head of the screw (214) as illustrated in FIG. 8.

FIG. 11 is a perspective view for illustrating a screwdriver for use in surgery in connection with the cervical spine fixator according to the third embodiment of the present invention. The distal end of the screwdriver (230) is formed with a crossed protruder (232) for being fitted into the crossed groove (214b) of the screw (214). The crossed protruder (232) is made of a convex curvature to be fitted into the concave curvature of the crossed groove (214b). The distal end of the screwdriver is formed with a straight protruder, which protrudes from the cross-section thereof The straight protruder may be of a convex curvature for being fitted into the concave curvature of the straight groove of the screw.

The screwdriver thus described slowly pushes the distal end of the screw head fixture member (216) and bends the distal end of the screw head fixture member (216) when the crossed protruder (232) of the screwdriver is driven into the crossed groove (214b) of the screw (214) while the screw (214) is fixed by the screw head fixture member (216). As a result, a separate tool is not necessary for deforming the screw head fixture member (216) when the screw (214) is removed, thus shortening the operating time and adding convenience to the surgical procedure.

As apparent from the foregoing, there is an advantage in the cervical spine fixator and a screwdriver used therefor thus described according to the present invention in that it is easy to manufacture, and the plate is reduced in thickness, thus eliminate the feeling of a foreign substance after a surgical operation.

There is another advantage in that it is easy to perform surgery with the fixator and it is possible to shorten the operating time.

The foregoing description of the preferred embodiment of the present invention has been presented for the purpose of illustration and description. It is not intended to be exhaustive or to limit the invention to the precise form disclosed, and modifications and variations are possible in light of the above teachings or may be acquired from practice of the invention. It is intended that the scope of the invention be defined by the claims appended hereto.

## Claims

1. A cervical spine fixator, comprising:
a plate (12, 212) for being adjoined to a cervical spine fixation region; and
a plurality of screws (14, 214) for insertion and fixation into a cervical spine and for being simultaneously coupled to said plate (12, 212), wherein an adjacent region in the plate (12, 212) is coupled with said screws (14, 214) and is fixed with a screw head fixture member (16, 216) having a protruder (16a), which is bent under the effect of pressure by a screw head when said screw (14, 214) is inserted and the protruder (16a) returns to its original position when said screw head is completely accommodated into the plate (12, 212) to be hitched by said screw head, and said plate-(12, 212) is formed thereon with a receiving groove (22, 222) for accommodating said screw head and said screw head fixture member (16, 216) and said receiving groove (22, 222) is formed with openings (22a, 222a) for insertion of said screws (14, 214) and a recess for receiving said screw head,
**characterised** that,
the distal tip ends of said protruder (16a) are formed with a hitching protruder (216a) and said recess of said plate (12, 212) is formed with a hitching hole (222e) for passing through the thickness of said plate (12, 212) for accommodating said hitching protruder (216a) and the opposite end of the screw head fixture member (16, 216) is fixed to said plate (12, 212).

2. The fixator as defined in claim 1, wherein said screw head fixture member (16, 216) is an elastic plate (12, 212) protrusively formed with said protruder.

3. The fixator as defined in claim 1, wherein said screw head fixture member (16, 216) is a U-shaped elastic ring (116a) whose both ends functioning as protruders.

4. The fixator as defined in claim 2 or 3, wherein the distal tip ends of said protruder (16, 216) or said elastic ring (116) are bent towards each other.

5. The fixator as defined in claim 1, wherein the mid-section of said plate (12, 212) is formed with a window-shaped hole (212a) to observe a cage between the vertebrae during surgery, and the window-shaped hole (212a) is formed with a screw thread (212b) into which a screw is threaded for fixing said cage.

6. The fixator as defined in any one of claims 1, 2 or 3, wherein said screw head is formed with a cylindrical incline so that said protruder (16a, 216a) or said U-shaped elastic ring (116) can smoothly slide to be elastically deformed under the effect of pressure when said screws (14, 214) are inserted into said plate.

7. The fixator as defined in any one of claims 1, 2 or 3, wherein said protruder of said screw head fixture member (16, 216) or the opposite end of the distal end at said U-shaped elastic ring (116) is fixed to said plate (12, 212) by rivets (18, 218) or by welding.

8. The fixator as defined in any one of claims 1, 2 or 3, wherein the upper surface of the screw head is grooved in the shape of a straight line or a cross, and the grooved surface is deepened towards the center thereof to form a concaved curvature so that said protruder (16a) or said U-shaped elastic ring (116) can be deformed under the effect of pressure by the tip end (232) of a screwdriver (230) when the screws are installed or removed.

9. A kit comprising the fixator according to claim 8 and a screwdriver for rotating the screws, wherein the distal tip end of said screwdriver (230) for rotating said screws (14, 214) is protrusively formed with a protruder in the shape of a straight line or a cross for insertion into the groove, and said protruder is formed in a convex shape for being fitted into the concaved curvature.

## Patentansprüche

1. Halswirbelstabilisierungsvorrichtung, umfassend:
eine Platte (12, 212) zur Anbringung an einem Halswirbelstabilisierungsbereich und
eine Vielzahl von Schrauben (14, 214) zum Einführen und Befestigen in einen Halswirbel und um gleichzeitig mit der Platte (12, 212) verbunden zu sein,
wobei ein benachbarter Bereich in der Platte (12, 212) mit den Schrauben (14, 214) verbunden ist und mit einem Schraubenkopf-Befestigungsteil (16, 216) stabilisiert ist, das einen Überstand (16a) aufweist, der unter dem Einfluss des Drucks durch einen Schraubenkopf gebogen wird, wenn die Schraube (14, 214) eingeführt wird, und
wobei der Überstand (16a) in seine Ausgangsposition zurückkehrt, wenn der Schraubenkopf vollständig in die Platte (12, 212) aufgenommen ist, um durch den Schraubkopf festgelegt zu werden, und wobei die Platte (12, 212) auf sich mit einer Aufnahmevertiefung (22, 222) gebildet ist, um den Schraubenkopf und das Schraubenkopf-Befestigungsteil (16, 216) aufzunehmen, und wobei die Aufnahmevertiefung (22, 222) mit Öffnungen (22a, 222a) versehen ist für das Einführen der Schrauben (14, 214),
und mit einer Aussparung für die Aufnahme des Schraubenkopfs aufzunehmen,
**dadurch gekennzeichnet, dass**
die distalen Endspitzen des Überstands (16a) mit einem festlegenden Überstand (216a) gebildet sind und die Aussparung der Platte (12,212) mit einem durch die Stärke der Platte (12.212) reichenden Festlegungsloch (222e) versehen ist für das Aufnehmen des festlegenden Überstands (216a), wobei das abgewandte Ende des Schraubenkopf-Befestigungsteils (16, 216) an der Platte (12,212) befestigt ist.

2. Stabilisierungsvorrichtung nach Anspruch 1, wobei das Schraubenkopf-Befestigungsteil (16, 216) eine elastische Platte (12,212) ist, die Überstand bildend mit dem Überstand gebildet ist.

3. Stabilisierungsvorrichtung nach Anspruch 1, wobei das Schraubenkopf-Befestigungsteil (16, 216) ein U-förmiger elastischen Ring (116a) ist, dessen beide Enden als Überstände dienen.

4. Stabilisierungsvorrichtung nach Anspruch 2 oder 3, wobei die distalen EndSpitzen des Überstands (16,216) oder des elastischen Rings (116) zueinander hingebogen sind.

5. Stabilisierungsvorrichtung nach Anspruch 1, wobei der Mittelbereich der Platte (12,212) mit einem fensterförmigen Loch (212a) versehen ist, um einen Käfig zwischen den Wirbeln während des Operierens zu beobachten, und wobei das fensterförmige Loch (212a) mit einem Schraubgewinde (212b) geformt ist, in das eine Schraube gedreht wird, um den Käfig zu befestigen.

6. Stabilisierungsvorrichtung nach irgend einem der Ansprüche 1, 2 oder 3, wobei der Schraubenkopf mit einer zylindrischen Verjüngung versehen ist, so dass der Überstand (16a, 216a) oder der U-förmige elastische Ring (116) reibungsarm gleiten Kann, um unter dem Einfluss des Drucks elastisch deformiert zu werden, wenn die Schrauben (14,214) in die Platte eingeführt werden.

7. Stabilisierungsvorrichtung nach irgend einem der Ansprüche 1, 2 oder 3, wobei der Überstand des Schraubenkopf-Befestigungsteils (16, 216) oder das dem distalen Ende abgewandte Ende des U-förmigen elastischen Rings (116) durch Nieten (18,218) oder durch Verschweißen an der Platte (12,212) befestigt ist.

8. Stabilisierungsvorrichtung nach irgend einem der Ansprüche 1, 2 oder 3, wobei die Oberseite des Schraubenkopfes in der Form einer geraden Linie oder eines Kreuzes gerillt ist und die gerillte Oberseite zu ihrem Zentrum hin vertieft ist, um eine konkave Krümmung zu bilden, so dass der Überstand (16a) oder der U-förmige elastische Ring (116) unter dem Einfluss des Drucks durch die Endspitze (232) eines Schraubendrehers (230) verformt werden kann, wenn die Schrauben angebracht oder entfernt werden.

9. Set umfassend die Stabilisienmgsvorrichtung nach Anspruch 8 und einen Schaubendreher zum Drehen der Schrauben, wobei die distale Endspitze des Schraubendrehers (230) zum Drehen der Schrauben (14,214) mit einem Überstand in der Form einer geraden Linie oder eines Kreuzes zum Einführen in die Rille überstehend geformt ist, und wobei der Überstand in einer konvexen Form gebildet ist, um in die konkave Krümmung eingepasst zu werden.

## Revendications

1. Dispositif de stabilisation des vertèbres cervicales, comprenant :
une plaque (12, 212) à relier à une zone de fixation de la colonne cervicale ; et
une pluralité de vis (14, 214) à insérer et à fixer dans une colonne cervicale et à raccorder simultanément à ladite plaque (12, 212) dans laquelle une région adjacente dans la plaque (12, 212) est reliée avec lesdites vis (14, 214) et est fixée avec un élément de fixation de tête de vis (16, 216) ayant une saillie (16a) qui est pliée sous l'effet de la pression par une tête de vis quand ladite vis (14, 214) est insérée et la saillie (16a) revient dans sa position d'origine quand ladite tête de vis est totalement insérée dans la plaque (12, 212) de façon à être accrochée par ladite tête de vis, et sur ladite plaque (12, 212) est ménagée une rainure de réception (22, 222) pour supporter ladite tête de vis et ledit élément de fixation de tête de vis (16, 216) et ladite rainure de réception (22, 222) est formée avec des ouvertures (22a, 222a) permettant l'insertion desdites vis (14, 214) et une cavité pour recevoir ladite tête de vis,
**caractérisé en ce que**
les extrémités de pointe distale de ladite saillie (16a) sont formées avec une saillie d'accroche (216a) et ladite cavité de ladite plaque (12, 212) est formée avec un orifice d'accroche (222e) pour passer à travers l'épaisseur de ladite plaque (12, 212), afin de supporter ladite saillie d'accroche (216a) et l'extrémité opposée de l'élément de fixation de tête de vis (16, 216) est fixée à ladite plaque (12, 212).

2. Dispositif de stabilisation selon la revendication 1, dans lequel ledit élément de fixation de tête de vis (16, 216) est une plaque élastique (12, 212) formée de manière saillante avec ladite saillie.

3. Dispositif de stabilisation selon la revendication 1, dans lequel ledit élément de fixation de tête de vis (16, 216) est un anneau élastique en forme de U (116a) dont les deux extrémités fonctionnent comme des saillies.

4. Dispositif de stabilisation selon la revendication 2 ou 3, dans lequel les extrémités de pointe distale de ladite saillie (16, 216) ou dudit anneau élastique (116) sont pliées l'une vers l'autre.

5. Dispositif de stabilisation selon la revendication 1, dans lequel le centre de la section de ladite plaque (12, 212) présente un orifice en forme de fenêtre (212a) pour observer une cage entre les vertèbres pendant la chirurgie, et l'orifice en forme de fenêtre (212a) est formé avec un filet de vis (212b) dans lequel une vis est filetée pour la fixation de ladite cage.

6. Dispositif de stabilisation selon l'une quelconque des revendications 1, 2 ou 3, dans lequel ladite tête de vis est formée d'une déclivité cylindrique, de sorte que ladite saillie (16a, 216a) ou ledit anneau élastique en forme de U (116) peut régulièrement glisser pour être élastiquement déformé sous l'effet de la pression quand lesdites vis (14, 214) sont insérées dans ladite plaque.

7. Dispositif de stabilisation selon l'une quelconque des revendications 1, 2 ou 3, dans lequel ladite saillie dudit élément de fixation de tête de vis (16, 216) ou l'extrémité opposée de l'extrémité distale audit anneau élastique en forme de U (116) est fixée à ladite plaque (12, 212) par des rivets (18, 218) ou par soudage.

8. Dispositif de stabilisation selon l'une quelconque des revendications 1, 2 ou 3, dans lequel la surface supérieure de la tête de vis est rainurée en forme d'une ligne droite ou d'une croix, et la surface rainurée est plus profonde vers le centre de celle-ci, afin de former une courbure concave de sorte que ladite saillie (16a) ou ledit anneau élastique en forme de U (116) puisse être déformé, sous l'effet de la pression par l'extrémité de pointe (232) d'un tournevis (230) quand les vis sont installées ou enlevées.

9. Kit comprenant le dispositif de stabilisation selon la revendication 8 et un tournevis pour tourner les vis, dans lesquels l'extrémité de pointe distale dudit tournevis (230) pour tourner lesdites vis (14, 214) est formée de manière saillante avec une saillie en forme de ligne droite ou de croix pour permettre l'insertion dans la rainure, et ladite saillie est réalisée avec une forme convexe pour être adaptée à la courbure concave.
